(19) Europäisches Patentamt
European Patent Office
Office européen des brevets

(11) EP 4 442 834 A1

(12) **EUROPEAN PATENT APPLICATION**
published in accordance with Art. 153(4) EPC

(43) Date of publication:
**09.10.2024 Bulletin 2024/41**

(21) Application number: **22901747.0**

(22) Date of filing: **30.11.2022**

(51) International Patent Classification (IPC):
***C12P 19/12*** *(2006.01)* ***C12P 19/14*** *(2006.01)*
***C12N 9/42*** *(2006.01)* ***C07H 15/04*** *(2006.01)*
***C12N 1/14*** *(2006.01)*

(52) Cooperative Patent Classification (CPC):
**C07H 15/04; C12N 1/14; C12N 9/2434; C12P 19/12; C12P 19/14**

(86) International application number:
**PCT/KR2022/019160**

(87) International publication number:
**WO 2023/101389 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2021 KR 20210168329**
**30.11.2021 KR 20210168330**

(71) Applicant: **CJ CheilJedang Corporation**
**Seoul 04560 (KR)**

(72) Inventors:
- **LEE, Sungkyun**
**Seoul 04560 (KR)**
- **KIM, Minhoe**
**Seoul 04560 (KR)**
- **PARK, Jung Won**
**Seoul 04560 (KR)**
- **HONG, Hyeongpyo**
**Seoul 04560 (KR)**
- **KIM, Taekbeom**
**Seoul 04560 (KR)**

(74) Representative: **Reddie & Grose LLP**
**The White Chapel Building**
**10 Whitechapel High Street**
**London E1 8QS (GB)**

# (54) METHOD FOR PRODUCING DISACCHARIDE USING BETA-GLUCOSIDASE AND COFACTOR THEREOF AND COMPOSITION FOR INDUCING ENZYME PRODUCTION OF STRAIN OF GENUS TRICHODERMA COMPRISING PRODUCED DISACCHARIDE

(57) The present invention relates to a method for producing a disaccharide using beta-glucosidase and a cofactor thereof and a composition for inducing the enzyme production of a strain of the genus *Trichoderma,* the composition comprising the produced disaccharide. Particularly, when beta-glucosidase and a cofactor thereof, such as a manganese ion ($Mn^{2+}$), a magnesium ion ($Mg^{2+}$), a zinc ion ($Zn^{2+}$) or a copper ion ($Cu^{2+}$) as a divalent metal ion, are added to a glucose matrix, the production rate of the disaccharide increases, and the disaccharide can be obtained at a high concentration. In addition, a composition containing a high concentration of a disaccharide produced using the production method of the present invention has an excellent effect of inducing the enzyme production of a strain of the genus *Trichoderma,* and thus, the enzyme productivity of *Trichoderma* can be improved by applying a fermentation process utilizing a high concentration of the produced disaccharide.

EP 4 442 834 A1

## Description

## [TECHNICAL FIELD]

CROSS-REFERENCE TO RELATED APPLICATION(S)

This application claims priority benefit of Korean Patent Application No. 10-2021-0168329 filed on November 30, 2021 and Korean Patent Application No. 10-2021-0168330 filed on November 30, 2021 in the Korean Intellectual Property Office, the disclosures of which are incorporated herein by reference in their entirety.

The present invention relates to a method for producing a disaccharide using beta-glucosidase and a cofactor thereof and a composition for inducing the enzyme production of a strain of the genus *Trichoderma* comprising the produced disaccharide, and more specifically, to a method that can increase the production rate of disaccharides such as gentiobiose or sophorose from glucose by adding beta-glucosidase and a divalent metal ion as cofactor thereof, thereby obtaining disaccharides at high concentrations. In addition, the present invention relates to a composition for promoting enzyme production of a strain of the genus *Trichoderma* comprising a high content of the produced disaccharide and a method for producing an enzyme using the composition.

## [BACKGROUND ART]

Sophorose is a disaccharide having two molecules of D-glucopyranosyl 2-O-β bonds, which is a type of maltose type disaccharide.

Gentiobiose is a disaccharide made up of two D-glucose molecules linked together by a β-bond, which is also called amygdalose. It dissolves in water or methanol as a white crystalline solid, and when hydrolyzed with acid or β-glucosidase, two molecules of D-glucose are produced.

The disaccharides such as sophorose and gentiobiose are known as inducers for enzyme production of *Trichoderma reesei.*

One of the useful methods for producing the disaccharides is a method that utilizes a reverse hydrolysis reaction by an enzyme. A representative enzyme used in the method for oligosaccharide synthesis is beta-glucosidase. Beta-glucosidase is an enzyme that hydrolyzes the beta-1,4-glucosidic bond between two glucoses or between glucose and other substituted molecules to release glucose, which is a type of exocellulase for which specificity exists for matrixes of various beta D-glucoside families. However, the reverse hydrolysis reaction by an enzyme has a low conversion efficiency from monosaccharides to disaccharides due to the equilibrium being biased in the hydrolysis direction, which limits large-scale production.

It has been reported that cellulases produced by most microorganisms are inducible enzymes, and cellulose, cellobiose, lactose, sophorose, or the like are good inducing factors (Antonov et al. Microb Cell Fact (2016) 15:164).

It is known that the process by which fibrinolytic enzymes are synthesized is regulated by the induction process and catabolite repression. Specifically, it is known that sophorose, gentiobiose, or laminaribiose induce cellulase synthesis in a strain of *Penicillium purpurogenum* P-26 (APPLIED AND ENVIRONMENTAL MICROBIOLOGY, Jan. 1992, p. 106-110), and it is known that trehalose induces cellulase synthesis in a stain of *Clostridium papyrosolvens* CFR-703 (Process Biochemistry 37 (2001) 241-245). In addition, for the activity of cellulolytic enzymes, cellulase, which is also regulated by end product inhibition, is inhibited by cellobiose, and in the case of *Thermonospora fusca* and *Trichoderma reesei* strains, β-glucosidase is inhibited by glucose. The degree of synthesis and composition of cellulolytic enzymes synthesized as a result of such complicated regulatory actions vary greatly depending on the type of microorganism and the culture conditions.

## [DETAILED DESCRIPTION OF THE INVENTION]

### [Technical Problem]

It is an object of the present invention to provide a composition comprising 1-hydroxyglucitol-D-glucopyranoside or an isomer thereof.

It is another object of the present invention to provide a method for producing a composition for inducing enzyme production of a microorganism or for increasing enzyme activity of a microorganism, the method comprising 1) contacting glucose with beta-glucosidase; and 2) contacting the reaction product of step 1 with a divalent metal ion.

It is another object of the present invention to provide a composition for inducing the enzyme production of a strain of the genus *Trichoderma* comprising disaccharides of trehalose, isomaltose, gentiobiose, cellobiose, sophorose and maltose.

It is yet another object of the present invention to provide a method for producing an enzyme from a strain of

the genus *Trichoderma* using a composition for inducing enzyme production of a strain of the genus *Trichoderma* which comprises the disaccharide.

It is a further object of the present invention to provide use of a composition comprising high contents of disaccharides of trehalose, isomaltose, gentiobiose, cellobiose, sophorose and maltose for inducing enzyme production.

**[Technical Solution]**

Respective descriptions and embodiments disclosed herein may be applied to other descriptions and embodiments. That is, all combinations of various elements disclosed herein fall within the scope of the present invention. Additionally, the scope of the present invention should not be regarded as being limited by the specific description described below. Moreover, those of ordinary skill in the art may be able to recognize or confirm, using only conventional experimentation, many equivalents to the particular embodiments of the invention described herein. Further, it is also intended that these equivalents be included in the present invention.

In one embodiment, the present invention provides a method for producing a composition for inducing enzyme production of a microorganism or for increasing enzyme activity of a microorganism, the method comprising the steps of: 1) contacting glucose with beta-glucosidase; and 2) contacting the glucose or the reaction product of step 1 with a divalent metal ion.

The "glucose" in step 1) may be in the form of liquid glucose or an aqueous glucose solution, but is not limited thereto. Glucose of the present invention may be contained in a concentration of 65 to 77% w/w, 67 to 75% w/w, or 69 to 73% w/w.

The beta-glucosidase may be derived from the genus *Trichoderma.* Specifically, the beta-glucosidase of the present invention may be derived from *Trichoderma reesei.* More specifically, the beta-glucosidase of the present invention may be a polypeptide consisting of the amino acid sequence of SEQ ID NO: 1.

The microorganism means a microorganism that produces an enzyme, wherein the microorganism may be a microorganism that produces cellulase, xylanase, or phytase enzymes, or may be a microorganism that can produce all of cellulase, xylanase, and phytase enzymes, but is not limited thereto. In one embodiment, the microorganism may be a strain of the genus *Trichoderma,* and more specifically, a strain of *Trichoderma reesei.*

The beta-glucosidase may be 1,300 to 2,000 U/ml, 1,400 to 1,900 U/ml, 1,500 to 1,800 U/ml, or 1,550 to 1,740 U/ml, but is not limited thereto.

The divalent metal ion may be at least one selected from the group consisting of manganese ion ($Mn^{2+}$), magnesium ion ($Mg^{2+}$), zinc ion ($Zn^{2+}$), and copper ion ($Cu^{2+}$), or may be at least one selected from the group consisting of zinc ion ($Zn^{2+}$), and copper ion ($Cu^{2+}$), but is not limited thereto.

The "contacting step" of step 2) may be used interchangeably with the terms "standing step" or "reacting step" herein.

The "contact" of step 2) may be performed at 20 to 50 rpm, 25 to 45 rpm, or 28 to 42 rpm, but is not limited thereto.

The "contact" of step 2) may be performed at pH 3.0 to 7.0, pH 3.5 to 6.5, pH 4.0 to 6.0, or pH 4.5 to 5.5, but is not limited thereto.

The "contact" of step 2) may be performed at 50°C to 70°C, 55°C to 65°C, or 57°C to 63°C, but is not limited thereto.

The "contact" of step 2) may be performed for 5 days or more, may be performed for 6 days or more, may be performed for 7 days or more, may be performed for 5 to 22 days, or may be performed for 6 to 21 days, but is not limited to thereto.

The produced composition may include any one or more selected from the group consisting of trehalose, isomaltose, cellobiose, maltose, gentiobiose, sophorose, 1-hydroxyglucitol-D-glucopyranoside, and an isomer thereof, but is not limited to thereto.

The produced composition may include gentiobiose and sophorose, but is not limited to thereto.

The produced composition has an excellent effect of inducing the enzyme production of a stain of the genus *Trichoderma* or the fermentation activity of the produced enzyme.

Further, the method for producing the composition of the present invention may further comprise a step of performing chromatography containing divalent cations.

The chromatography of the present invention may be a simulated moving bed (SMB) chromatography.

In the chromatography of the present invention, the divalent cation may be contained in the form of a column packed with a cation exchange resin.

In the chromatography of the present invention, the divalent cation may be any one or more of calcium ions, barium ions, and strontium ions.

The simulated moving bed chromatography of the present invention may include 8 or more columns.

The simulated moving bed chromatography of the present invention may be one in which elution is performed with water at 50°C to 70°C. Specifically, the elution in the present invention can be performed with water at 55 to 65°C

or 58 to 62°C.

In another embodiment, the present invention provides (1S)-1-hydroxyglucitol-D-glucopyranoside having the structure of the following Chemical Formula 1.

[Chemical Formula 1]

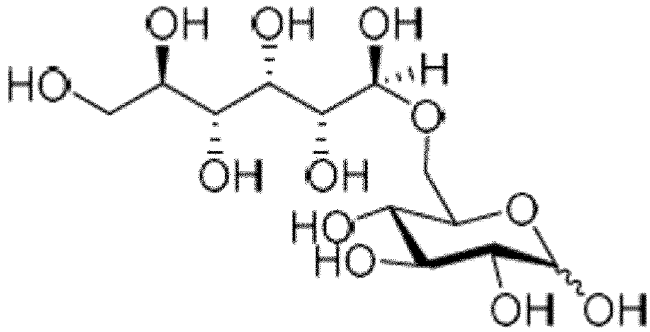

In another embodiment, the present invention provides 1-epi-1-hydroxyglucitol-D-glucopyranoside having the structure of the following Chemical Formula 2.

[Chemical Formula 2]

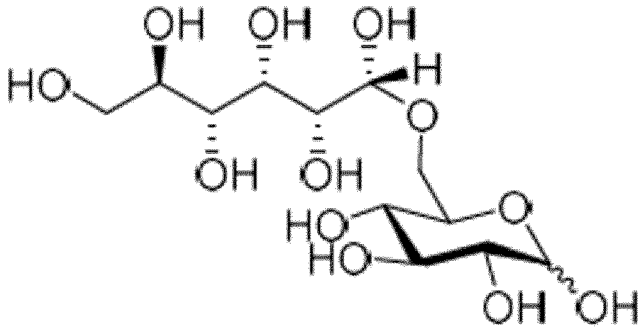

In another embodiment, the present invention provides a composition comprising 1-hydroxyglucitol-D-glucopyranoside or an isomer thereof.

The composition may induce the enzyme production of a microorganism or increase the activity of an enzyme produced by a microorganism.

Specifically, the 1-hydroxyglucitol-D-glucopyranoside or an isomer thereof may be (1S)-1-hydroglucitol-D-glucopyranoside or 1-epi-1-hydroglucitol-D-glucopyranoside.

In one embodiment, the present invention provides a composition for inducing enzyme production, which comprises a high content of disaccharide.

The disaccharide may be any one or more selected from the group consisting of trehalose, isomaltose, gentiobiose, cellobiose, sophorose, maltose, 1-hydroxyglucitol-D-glucopyranoside and an isomer thereof.

In one embodiment, the present invention provides a composition for inducing the enzyme production of a microorganism, which comprises trehalose, isomaltose, gentiobiose, cellobiose, sophorose and maltose.

As used herein, the "inducing enzyme production" may mean inducing the amount of enzyme produced from a microorganism, and the "inducing enzyme production" can be confirmed by measuring the fermentation activity of the produced enzyme.

The microorganism may be a strain of the genus *Trichoderma,* may be a strain of *Trichoderma reesei,* and more specifically, may be a strain of *Trichoderma reesei* QM6a, but is not limited thereto.

The enzyme may be an enzyme produced by a strain of the genus *Trichoderma,* may be an enzyme produced by a strain of *Trichoderma reesei,* and more specifically, may be an enzyme produced by a strain of *Trichoderma reesei* QM6a, but is not limited thereto.

The enzyme may be cellulase, xylanase, or phytase, and preferably xylanase.

The composition for inducing the enzyme production of a strain of the genus *Trichoderma* may further comprise

1-hydroxyglucitol-D-glucopyranoside or an isomer thereof.

The 1-hydroxyglucitol-D-glucopyranoside or an isomer thereof may be (1S)-1-hydroglucitol-D-glucopyranoside or 1-epi-1-hydroglucitol-D-glucopyranoside.

The 1-hydroxyglucitol-D-glucopyranoside or an isomer thereof may be prepared by a method for producing a disaccharide, which comprises 1) contacting glucose with beta-glucosidase, and 2) contacting the glucose or the reaction product of step 1 with a divalent metal ion.

The composition may be low in monosaccharide content and high in disaccharide content.

The composition may have a higher disaccharide content than the monosaccharide content contained in the composition, and the increase in the disaccharide content may be achieved by Simulated Moving Bed (SMB) chromatography.

The content of monosaccharide contained in the composition may be 45% by weight or less, 40% by weight or less, 35% by weight or less, 30% by weight or less, 20% by weight to 45% by weight, 25% by weight to 45% by weight, 20% by weight to 40% by weight, 25% by weight to 40% by weight, 20% by weight to 35% by weight, 25% by weight to 35% by weight, 20% by weight to 30% by weight, or 25% by weight to 30% by weight based on the total weight of the composition.

The content of disaccharide contained in the composition may be 27% by weight or more, 30% by weight or more, 33% by weight or more, 36% by weight or more, 39% by weight or more, 27% by weight to 50% by weight, 30% by weight to 50% by weight, 33% by weight to 50% by weight, 36% by weight to 50% by weight, 39% by weight to 50% by weight, 27% by weight to 45% by weight, 30% by weight to 45% by weight, 33% by weight to 45% by weight, 36% by weight to 45% by weight, 39% by weight to 45% by weight, 27% by weight to 42% by weight, 30% by weight to 42% by weight, 33% by weight to 42% by weight, 36% by weight to 42% by weight, 39% by weight to 42% by weight, 27% by weight to 40% by weight, 30% by weight to 40% by weight, 33% by weight to 40% by weight, 36% by weight to 40% by weight, or 39% by weight to 40% by weight, based on the total weight of the composition.

The content of trehalose contained in the composition may be 2.5% by weight or more, 3% by weight or more, 4% by weight or more, 2.5% by weight to 6% by weight, 3% by weight to 6% by weight, 4% by weight to 6% by weight, 2.5% by weight to 5% by weight, 3% by weight to 5% by weight, or 4% by weight to 5% by weight, based on the total weight of the composition.

The content of isomaltose contained in the composition may be 4.5% by weight or more, 5% by weight or more, 4.5% by weight to 7% by weight, 5% by weight to 7% by weight, 4.5% by weight to 6% by weight, or 5% by weight to 6% by weight, based on the total weight of the composition.

The content of gentiobiose contained in the composition may be 15% by weight or more, 17% by weight or more, 19% by weight or more, 21% by weight or more, 15% by weight to 28% by weight, 17% by weight to 28% by weight, 19% by weight to 28% by weight, 21% by weight to 28% by weight, 15% by weight to 25% by weight, 17% by weight to 25% by weight, 19% by weight to 25% by weight, 21% by weight to 25% by weight, 15% by weight to 23% by weight, 17% by weight to 23% by weight, 19% by weight to 23% by weight, or 21% by weight to 23% by weight, based on the total weight of the composition.

The content of cellobiose contained in the composition may be 1.9% by weight or more, 2.0% by weight or more, 2.1% by weight or more, 2.2% by weight or more, 1.9% by weight to 3.0% by weight, 2.0% by weight to 3.0% by weight, 2.1% by weight to 3.0% by weight, 2.2% by weight to 3.0% by weight, 1.9% by weight to 2.7% by weight, 2.0% by weight to 2.7% by weight, 2.1% by weight to 2.7% by weight, 2.2% by weight to 2.7% by weight, 1.9% by weight to 2.5% by weight, 2.0% by weight to 2.5% by weight,2.1% by weight to 2.5% by weight, or 2.2% by weight to 2.5% by weight, based on the total weight of the composition.

The content of sophorose contained in the composition may be 1.4% by weight or more, 1.5% by weight or more, 1.6% by weight or more, 1.7% by weight or more, 1.4% by weight to 3.0% by weight, 1.5% by weight to 3.0% by weight, 1.6% by weight to 3.0% by weight, 1.7% by weight to 3.0% by weight, 1.4% by weight to 2.5% by weight, 1.5% by weight to 2.5% by weight, 1.6% by weight to 2.5% by weight, 1.7% by weight to 2.5% by weight, 1.4% by weight to 2.0% by weight, 1.5% by weight to 2.0% by weight, 1.6% by weight to 2.0% by weight, or 1.7% by weight to 2.0% by weight, based on the total weight of the composition.

The content of maltose contained in the composition may be 2.6% by weight or more, 3.0% by weight or more, 3.4% by weight or more, 3.7% by weight or more, 2.6% by weight to 5.0% by weight, 3.0% by weight to 5.0% by weight, 3.4% by weight to 5.0% by weight, 3.7% by weight to 5.0% by weight, 2.6% by weight to 4.6% by weight, 3.0% by weight to 4.6% by weight, 3.4% by weight to 4.6% by weight, 3.7% by weight to 4.6% by weight, 2.6% by weight to 4.2% by weight, 3.0% by weight to 4.2% by weight, 3.4% by weight to 4.2% by weight, 3.7% by weight to 4.2% by weight, 2.6% by weight to 3.9% by weight, 3.0% by weight to 3.9% by weight, 3.4% by weight to 3.9% by weight, or 3.7% by weight to 3.9% by weight, based on the total weight of the composition.

The composition may further comprise an oligomer.

The content of an oligomer contained in the composition may be 29% by weight to 35% by weight, 29% by weight to 34% by weight, 29% by weight to 33% by weight, 30% by weight to 35% by weight, 30% by weight to 34% by

weight, 30% by weight to 33% by weight, 31% by weight to 35% by weight, 31% by weight to 34% by weight, 32% by weight to 35% by weight, or 32% by weight to 34% by weight, based on the total weight of the composition, but is not limited thereto.

In one embodiment described herein, it was confirmed that the composition (SMB separated liquid) having a low monosaccharide content and a high disaccharide content exhibits superior enzymatic fermentation activity compared to a composition (TGS) having a high monosaccharide content and a low disaccharide content. Therefore, the composition containing high contents of trehalose, isomaltose, gentiobiose, cellobiose, sophorose and maltose can be used to induce enzyme production of a microorganism.

**[Advantageous Effects]**

The present invention relates to a method for producing a disaccharide using beta-glucosidase and a cofactor thereof and a composition for inducing the enzyme production of a strain of the genus *Trichoderma,* the composition comprising the produced disaccharide. Particularly, when beta-glucosidase and a cofactor thereof, such as a manganese ion ($Mn^{2+}$), a magnesium ion ($Mg^{2+}$), a zinc ion ($Zn^{2+}$) or a copper ion ($Cu^{2+}$) as a divalent metal ion, are added to a glucose matrix, the production rate of the disaccharide increases, and the disaccharide can be obtained at a high concentration. In addition, a composition containing a high concentration of a disaccharide produced using the production method of the present invention has an excellent effect of inducing the enzyme production of a strain of the genus *Trichoderma,* and thus, the enzyme productivity of *Trichoderma* can be improved by applying a fermentation process utilizing a high concentration of the produced disaccharide.

**[DETAILED DESCRIPTION OF THE EMBODIMENTS]**

Hereinafter, the present invention will be described in more detail by way of Examples. However, these Examples are given for illustrative purposes only, and the scope of the invention is not intended to be limited to or by these Examples.

**Example 1. Preparation of disaccharide composition**

Glucose powder with a purity of 95% or more (hydrous crystalline glucose, Daejung Chemicals & Metals) was dissolved using steam to prepare 40 kg of a glucose solution with a concentration of 71% w/w. 0.3 kg of β-glucosidase (SEQ ID NO: 1) was added at a concentration of 1,650 U/ml to the prepared glucose solution, allowed to stand at 60°C for 7 days to 21 days to prepare a composition comprising disaccharides (Treated Glucose Syrup, TGS).

**Example 2. Preparation of disaccharide composition using the addition of divalent metal cation**

0.3 kg of β-glucosidase (same as in Example 1) was added to the glucose solution of Example 1 at a concentration of 1,650 U/ml, and 1 mM of divalent ions [manganese ion ($Mn^{2+}$), magnesium ion ($Mg^{2+}$), zinc ion ($Zn^{2+}$), and copper ion ($Cu^{2+}$) each] was further added thereto, and allowed to stand at 60°C for 7 days to 21 days to prepare a composition containing disaccharides.

**Example 3. Confirmation of the disaccharide concentration in the disaccharide composition and increase/decrease rate of disaccharide due to the addition of divalent metal cation**

In the compositions prepared in Examples 1 and 2, the production concentration (g/L) and increase/decrease rate (%) of the disaccharides, i.e. trehalose, isomaltose, maltose, cellobiose, gentiobiose, and sophorose were measured, and the results are shown in Table 1 and Table 2 below. The production concentration was analyzed using a Bio-LC system (Dionex ICS-3000, Sunnyvale, CA, United States) equipped with an electrochemical detector and a CarboPac PAI column.

[Table 1]

| Standing period (day) | Added ion | Concentration (g/L) | | | | | | |
|---|---|---|---|---|---|---|---|---|
| | | Trehalose | Isomaltose | Maltose | Cellobiose | Gentiobiose | Sophorose | Total |
| 7 | Not added | 21.9 | 8 | 16.6 | 14.4 | 58.4 | 6.9 | 65.3 |
| | $Mn^{2+}$ | 21.7 | 8.6 | 16.8 | 14.5 | 69.5 | 11.6 | 81.1 |
| | $Mg^{2+}$ | 21.1 | 6.7 | 13.7 | 13.1 | 64.3 | 10.8 | 75.1 |
| | $Zn^{2+}$ | 22.2 | 7.3 | 15.8 | 14.8 | 69.9 | 12.6 | 82.5 |
| | $Cu^{2+}$ | 19.4 | 8 | 18.7 | 15.1 | 71.7 | 13.3 | 85 |
| 14 | Not added | 11.4 | 9.3 | 12.1 | 13.1 | 70.6 | 12.7 | 83.3 |
| | $Mn^{2+}$ | 14.6 | 12.4 | 16.1 | 15.4 | 78 | 16.5 | 94.5 |
| | $Mg^{2+}$ | 15.3 | 10.6 | 14.6 | 15.6 | 69.4 | 17.4 | 86.8 |
| | $Zn^{2+}$ | 14.3 | 9.8 | 14.3 | 14.9 | 81 | 16.4 | 97.3 |
| | $Cu^{2+}$ | 12.6 | 10.3 | 15.9 | 15.2 | 82.1 | 17 | 99.1 |
| 21 | Not added | 15 | 14.1 | 15.2 | 15.3 | 82.7 | 15.9 | 98.6 |
| | $Mn^{2+}$ | 14.1 | 15.7 | 17.1 | 15.5 | 86.5 | 23.6 | 110.2 |
| | $Mg^{2+}$ | 14.8 | 13.2 | 15.4 | 15.4 | 74.6 | 23.1 | 97.7 |
| | $Zn^{2+}$ | 14.5 | 12.5 | 15.2 | 15 | 92 | 23.4 | 115.4 |
| | $Cu^{2+}$ | 13 | 12.4 | 16.8 | 15.5 | 92.4 | 24.3 | 116.7 |

[Table 2]

| Standing period (day) | Added ion | Increase/decrease rate (%) | | |
|---|---|---|---|---|
| | | Gentiobiose | Sophorose | Total |
| 7 | Not added | 0 | 0 | 0 |
| | $Mn^{2+}$ | 18.9 | 67.8 | 24.1 |
| | $Mg^{2+}$ | 10 | 56.8 | 15 |
| | $Zn^{2+}$ | 19.7 | 82.8 | 26.3 |
| | $Cu^{2+}$ | 22.8 | 92.2 | 30.1 |
| 14 | Not added | 0 | 0 | 0 |
| | $Mn^{2+}$ | 10.5 | 29.2 | 13.4 |
| | $Mg^{2+}$ | -1.6 | 36.5 | 4.2 |
| | $Zn^{2+}$ | 14.7 | 28.4 | 16.8 |
| | $Cu^{2+}$ | 16.3 | 33.5 | 18.9 |

(continued)

| Standing period (day) | Added ion | Increase/decrease rate (%) | | |
|---|---|---|---|---|
| | | Gentiobiose | Sophorose | Total |
| 21 | Not added | 0 | 0 | 0 |
| | $Mn^{2+}$ | 4.6 | 48.7 | 11.7 |
| | $Mg^{2+}$ | -9.8 | 45.3 | -0.9 |
| | $Zn^{2+}$ | 11.3 | 47.1 | 17 |
| | $Cu^{2+}$ | 11.7 | 53.0 | 18.4 |

**Example 4. Separation of high-purity polysaccharide** using **SMB chromatography**

To remove monosaccharide from the composition prepared in Example 2, simulated moving bed (SMB) chromatography was used. For the SMB chromatography, a Sequential Simulated Moving-bed System (Novasep, France) was used, wherein the system included eight columns connected in series (each column has a height of 95 cm and a diameter of 2.5 cm), a feed pump, a recirculation pump, an eluent pump, a heat exchanger, and a valve for flow control. SMB chromatography operating conditions are shown in Table 3 below, and the results of the separation experiment through SMB chromatography are shown in Table 4 below. The recovery rate was measured by comparing the amount of disaccharide and trisaccharide of raffinate with the amount of disaccharide and trisaccharide injected into the mixture (TGS) through SMB chromatography, and calculating the recovery rate using the following Equation.

$$\text{Recovery}(\%) = \text{Raffinate (sugar content)}/\text{Feed (sugar content)} \times 100$$

* Sugar content = the total amount of disaccharide and polysaccharide

[Table 3]

| Feeds | Composition prepared in Example 2 |
|---|---|
| Concentration | 60%(w/w) |
| Cation exchange resin | XA2004/32Ca |
| Column size and number | 25 mm X 950 mm, 8ea |
| Eluent | $H_2O$ |
| Eluent temperature | 60°C |
| Flow rate | mL/min |
| Feed | 1.2 |
| Water | 7 |
| Extract | 5.9 |
| Raffinate | 2.3 |

[Table 4]

| Sample name | HPLC Purity (%) | |
|---|---|---|
| | Disaccharide and Trisaccharide | Monosaccharide |
| SMB Feed | 36.1 | 63.9 |
| Raffinate | 81~89 | 10~19 |
| Extract | 3.9 | 96.1 |

As a result of the separation experiment using SMB chromatography, it was confirmed that the purity of disaccharide and trisaccharide in the raffinate fraction using calcium ions is 81-89%, and the recovery rate is 89%, as shown in Table 4.

**Example 5. NMR analysis of the prepared disaccharide component**

Acetylation was performed for NMR analysis on the composition prepared in Example 2. The reagents used were acetic anhydride (extra pure, Junsei Chemical Co.) and zinc chloride (Kanto Chemical Co.), and equipment conditions for separation and analysis of acetylated samples are shown in Table 5 below.

[Table 5]

| | |
|---|---|
| Equipment | Agilent technologies 1200 series |
| Column | YMC C18 (10 X 250mm, 5um) |
| Eluent | 60% methanol |
| Flow rate | 2mL/min |
| RI cell temperature | 35°C |

As a result, the previously known sugars, i.e. trehalose, isomaltose, gentiobiose, cellobiose, maltose, sophorose and maltose were confirmed in the analytical sample. Further, it was confirmed that a new sugar, i.e. (1S)-1-hydroxyglucitol-D-glucopyranoside or 1-epi-1-hydroglucitol-D-glucopyranoside exists in the analytical sample.

**Example 6. Analysis of sugar content of glucose solution and SMB separated liquid with concentrated disaccharide in glucose solution**

The following experiment was performed to analyze the sugar content of the TGS prepared in Example 2 and its SMB separated liquid. The SMB separated liquid is the raffinate fraction of Example 4 obtained by subjecting the disaccharide composition prepared in Example 2 to SMB chromatography

Specifically, the monosaccharide and disaccharide concentrations are shown in Table 6 below by analyzing the sugar content using a Bio-LC system (Dionex ICS-3000, Sunnyvale, CA, United States) equipped with an electrochemical detector and a CarboPac PAI column. The column was eluted with 0.1 M NaOH (0-5 min) at 30°C, followed by a linear gradient of sodium acetate (0-0.2 M) at 1 mL/min over 5-35 min.

[Table 6]

| Category | | TGS (Glucose enzyme treatment liquid) | | SMB separated liquid (TGS concentrated) | |
|---|---|---|---|---|---|
| Degree of polymerizatio n | Sugars | g/L | % | g/L | % |
| DP1 | Glucose | 213.5 | 44.81 | 125 | 27.36 |
| | Fructose | 1.3 | 0.27 | 3.1 | 0.68 |
| | sum | 214.8 | 45.08 | 128.1 | 28.04 |
| DP2 | Trehalose | 11.8 | 2.48 | 20.3 | 4.44 |
| | Isomaltose | 19.2 | 4.03 | 25 | 5.47 |
| | Gentiobiose | 67.5 | 14.17 | 99.5 | 21.78 |
| | Cellobiose | 8.9 | 1.87 | 10.1 | 2.21 |
| | Sophorose | 6.2 | 1.3 | 7.9 | 1.73 |
| | Maltose | 12 | 2.52 | 17.1 | 3.74 |
| | Sum | 125.6 | 26.36 | 179.9 | 39.37 |
| DP3 or more | oligomer | 136.1 | 28.56 | 148.9 | 32.59 |

(continued)

| Category | | TGS | | SMB separated liquid | |
|---|---|---|---|---|---|
| | | (Glucose enzyme treatment liquid) | | (TGS concentrated) | |
| Total | | 476.5 | 100 | 456.9 | 100 |

As a result, as shown in Table 6, the monosaccharide content of TGS was found to be 45.08%, and that of the SMB separated liquid was found to be 28.04%, confirming that the monosaccharide content of the SMB separated liquid was lower than that of TGS. In addition, in the case of disaccharide content, TGS was found to be 26.36%, and SMB separated liquid was found to be 39.37%, confirming that the disaccharide content of SMB separated liquid was higher than that of TGS.

**Example 7. Confirmation of enzyme production induction effect due to the disaccharide content contained in the composition**

To confirm whether the SMB separated liquid was effective in inducing enzyme production of a strain of the genus *Trichoderma,* the fermentation activity of the enzyme was measured using TGS and SMB separated liquid as follows.

Specifically, TGS or SMB separated liquid was added to the medium, respectively, and the strain of the genus *Trichoderma* was diluted to a microbial cell concentration of 190 or 200 g/L. After culturing at pH 4 and 28°C for 168 hours, the acid cellulase enzyme activity was measured, and shown in Table 7 below.

More specific measurements of the enzyme fermentation activity are shown in Examples 7-1 to 7-3 below.

**Example 7-1. Preparation of enzyme producing strains**

As the enzyme producing strains, *Trichoderma reesei* QM6a strain (ATCC13631) capable of producing an acid cellulase (4-β-D-glucan 4-glucanohydrolase EC 3.2.1.4), xylanase (1,4-β-D-xylan xylanohydrolase, EC3.2.1.8), and phytase (3-phytases, EC 3.1.3.8, 6-phytases, EC 3.1.3.26, and 5-phytases, EC 3.1.3.72) was prepared. Each strain was grown on potato dextrose agar plates at 30°C for 5 days to form spores. After forming spores, spores of each strain were resuspended in sterile NaCl solution (9 g/L), and 30% sterile glycerol was added. This mixture was stored in a 1.8 ml tube at -80°C.

**Example 7-2. Fed-batch culture of enzyme-producing strain**

The cultivation of the *Trichoderma reesei* QM6a strain producing each enzyme of Example 7-1 using fed-batch culture, was performed by feeding the carbon source in the feeding medium at a constant rate of 5.0 g/L per hour at the point when DO was increased by using DO as an indicator. The feeding medium was fed using a peristatic pump. At this time, the fermentation medium components were composed of carbohydrates 20.0 g/L, ammonium sulfate 5.0 g/L, and magnesium sulfate 1.5 g/L, calcium chloride 0.5 g/L, potassium phosphate dibasic 5.0 g/L, yeast extract 10.0 g/L, ferric sulfate 10.0 mg/L, cobalt chloride 4.0 mg/L, sodium molybdate 2.0 mg/L, boric acid 0.4 mg/L, and Tween 80 1.0 g/L. The feeding medium was composed of carbohydrate 600.0 g/L, yeast extract 6.0 g/L, and sodium chloride 1.0 g/L in which a weight ratio of carbon source and yeast extract was 1:0.01. As carbohydrates contained in the medium, TGS and SMB separated liquids were added in the activity experiments of each enzyme. Meanwhile, fermentation conditions were maintained at a culture temperature of 28°C, and pH was maintained at 4.0 using ammonia water. The stirring speed was adjusted to 600 rpm so that DO (dissolved oxygen) restrictions would not occur. At this time, the fungal culture was performed by inoculating 1 ml of the frozen spore mixture into 150 ml of medium in a 500 ml Erlenmeyer flask and pre-culturing it on a shaker at 28°C and 200 rpm for 72 hours, and then inoculating the culture solution with 10% (v/v).

**Example 7-3. Analysis of acid cellulase activity**

The fermentation broth obtained in Example 7-2 was centrifuged to remove T. *reesei* cells and other solid materials. The culture supernatant was appropriately diluted for enzyme analysis. All enzyme activities were expressed as specific activity using international units (IU) per mL of supernatant. 1 IU was defined as the amount of enzyme

required to liberate 1 μmol of D-glucose per minute under standard analysis conditions (5 mg/mL CMC, pH 4.8, 50°C).

Cellulases hydrolyze cellulose to produce monosaccharide and oligosaccharide under specific temperature and pH conditions. Oligosaccharide having reducing ends and monosaccharide having reducing groups undergo a color reaction with DNS reagent under high temperature conditions, and the color intensity of the reaction solution is proportional to the amount of reducing sugar produced by enzymatic hydrolysis, and the amount and reaction to reduce sugar production. The cellulase activity in the liquid is proportional, and the cellulase activity can be calculated by measuring the absorbance of the reaction solution by spectrophotometry.

[Table 7]

| Number | Experimental conditions | Cultivation time (hr) | Microbial cell concentration (g/L) | pH | Temperature (°C) | Enzyme activity (IU/mL) | Average activity (IU/mL) | Relative activity comparison (%) |
|---|---|---|---|---|---|---|---|---|
| 1 | TGS | 168 | 190 | 4 | 28 | 2728 | 2822.5 | 0 |
| 2 | TGS | 168 | 200 | 4 | 28 | 2917 | | |
| 3 | SMB separated liquid | 168 | 200 | 4 | 28 | 3973.333 | 3890 | 37.82108 |
| 4 | SMB separated liquid | 168 | 190 | 4 | 28 | 3806.667 | | |

As a result, as shown in Table 7, it was confirmed that when TGS was added, the average activity of acid cellulase was 2822.5 IU/mL; when SMB separated liquid was added, the average activity of acid cellulase was 3890 IU/mL, and when SMB separated liquid was added, the average activity of acid cellulase further increased by 37.8%.

**Example 8. Confirmation of the effect of inducing enzyme production by type of enzyme in the SMB separated liquid**

To confirm that the effect of SMB separated liquid on inducing enzyme production of a strain of the genus *Trichoderma* varies depending on the type of enzyme, TGS and SMB separated liquid were used to measure the fermentation activities of xylanase and phytase as follows.

Specifically, the TGS or SMB separated liquid was added to the medium, respectively, and the strain of the genus *Trichoderma* was diluted to a microbial cell concentration of 240 or 250 g/L. After culturing at pH 4 and 28°C for 168 hours, the enzyme activities of xylanase and phytase were measured, and are shown in Table 8 below.

More specifically, it was measured in the same manner as the strain preparation process and culture process described in Examples 7-1 and 7-2, and the analysis of each enzyme activity is shown in Examples 8-1 and 8-2 below.

**Example 8-1. Analysis of neutral xylanase activity**

The fermentation broth obtained in Example 7-2 was centrifuged to remove T. *reesei* cells and other solid materials. Culture supernatants were appropriately diluted for enzyme analysis. All enzyme activities were expressed as specific activity using international units (IU) per mL of supernatant. 1 IU was defined as the amount of enzyme required to liberate 1 μmol of D-xylose per minute under standard analysis conditions (1% xylan, pH 6.5, 50°C).

Under certain temperature and pH conditions, xylanase decomposes xylan into oligosaccharide and monosaccharide. Oligosaccharide having reduced ends and monosaccharide having reducing groups undergo a color reaction with DNS reagent. The color intensity of the reaction solution is proportional to the amount of reducing sugar produced by enzymatic hydrolysis, and the amount of reducing sugar produced is proportional to the activity of xylanase in the reaction solution. Therefore, the activity of xylanase can be calculated by a spectrophotometer.

**Example 8-2. Analysis of phytase activity**

The fermentation broth obtained in Example 7-2 was centrifuged to remove T. *reesei* cells and other solid materials. Culture supernatants were appropriately diluted for enzyme analysis. All enzyme activities were expressed as specific activity using international units (IU) per mL of supernatant. 1 IU was defined as the amount of enzyme required to liberate 1 μmol of inorganic phosphorus per minute in a sodium phytate solution at a concentration of 5.0 mmol/L per minute, which is the phytase activity unit under the conditions of 37°C and pH=5.5.

Under certain temperature and pH conditions, phytase completely hydrolyzes sodium phytate as a matrix to produce orthophosphoric acid and inositol derivatives. In acidic solutions, it can form a yellow compound with ammonium vanadium molybdate, which can be measured colorimetrically at a wavelength of 415 nm.

[Table 8]

| Enzyme | Experimental conditions | Culturing time (hr) | Microbial cell concentration (g/L) | pH | Temperature (°C) | Enzyme activity (IU/mL) | Relative activity comparation (%) |
|---|---|---|---|---|---|---|---|
| Neutral xylanase | TGS | 168 | 250 | 4 | 28 | 105376 | 0 |
| | SMB separated liquid | 168 | 240 | 4 | 28 | 144716 | 37.3 |
| Phytase | TGS | 168 | 240 | 4 | 28 | 25312 | 0 |
| | SMB separated liquid | 168 | 240 | 4 | 28 | 26668 | 5.4 |

As a result, as shown in Table 8, it was confirmed that when TGS was added, the average activity of neutral xylanase was 105,376 IU/mL; when SMB separated liquid was added, the average activity of neutral xylanase was

144,716 IU/mL; when SMB separated liquid was added, the fermentation activity of neutral xylanase further increased by 37.3%. In addition, when TGS was added, the average activity of phytase was 25,312 IU/mL; when SMB separated liquid was added, the average activity of phytase was 26,668 IU/mL, and when SMB separated liquid was added, the fermentation activity of phytase further increased by 5.4%.

## Claims

1. A composition comprising 1-hydroxyglucitol-D-glucopyranoside or an isomer thereof.

2. The composition according to claim 1, wherein the 1-hydroxyglucitol-D-glucopyranoside or an isomer thereof is (1S)-1-hydroglucitol-D-glucopyranoside or 1-epi-1-hydroxyglucitol-D-glucopyranoside.

3. The composition according to claim 1, wherein the composition further comprises any one or more selected from the group consisting of trehalose, isomaltose, gentiobiose, cellobiose, sophorose, and maltose.

4. The composition according to claim 1, wherein the composition induces the enzyme production of a microorganism or increases the activity of an enzyme produced by a microorganism.

5. The composition according to claim 4, wherein the microorganism is a strain of the genus *Trichoderma.*

6. The composition according to claim 4, wherein the microorganism is *Trichoderma reesei.*

7. The composition according to claim 4, wherein the enzyme is acid cellulase, xylanase or phytase.

8. The composition according to claim 1, wherein the composition has a disaccharide content higher than a monosaccharide content.

9. The composition according to claim 1, wherein the disaccharide content contained in the composition is 30% by weight or more based on the total weight of the composition.

10. The composition according to claim 1, wherein the monosaccharide content contained in the composition is 30% by weight or less based on the total weight of the composition.

11. A method for producing a composition for inducing enzyme production of a microorganism or for increasing enzyme activity of a microorganism, the method comprising the steps of:

    1) contacting glucose with beta-glucosidase; and
    2) contacting the glucose or the reaction product of step 1 with a divalent metal ion.

12. The method according to claim 11, wherein the beta-glucosidase is derived from the genus *Trichoderma.*

13. The method according to claim 11, wherein the beta-glucosidase is derived from *Trichoderma reesei.*

14. The method according to claim 11, wherein the divalent metal ion is at least one selected from the group consisting of manganese ion ($Mn^{2+}$), magnesium ion ($Mg^{2+}$), zinc ion ($Zn^{2+}$), and copper ion ($Cu^{2+}$).

15. The method according to claim 11, wherein the composition comprises any one or more selected from the group consisting of trehalose, isomaltose, cellobiose, maltose, gentiobiose, sophorose, 1-hydroxyglucitol-D-glucopyranoside and an isomer thereof.

16. The method according to claim 11, wherein the microorganism is a strain of the genus *Trichoderma.*

17. The method according to claim 11, wherein the method further comprises performing chromatography containing divalent cations.

18. The method according to claim 17, wherein the chromatography is a simulated moving bed (SMB) chromatography.

## INTERNATIONAL SEARCH REPORT

International application No.
**PCT/KR2022/019160**

**A. CLASSIFICATION OF SUBJECT MATTER**

**C12P 19/12**(2006.01)i; **C12P 19/14**(2006.01)i; **C12N 9/42**(2006.01)i; **C07H 15/04**(2006.01)i; **C12N 1/14**(2006.01)i

According to International Patent Classification (IPC) or to both national classification and IPC

**B. FIELDS SEARCHED**

Minimum documentation searched (classification system followed by classification symbols)

C12P 19/12(2006.01); C12N 1/14(2006.01); C12N 1/38(2006.01); C12N 9/42(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 1-하이드록시글루시톨-D-글루코피라노사이드(1-hydroxyglucitol-D-glucopyranoside), 베타-글루코시다아제(β-glucosidase), 트리코데르마 레세이(Trichoderma reesei), 2가 금속(divalent metal), 이당류(disaccharide), 셀룰라아제(cellulase)

**C. DOCUMENTS CONSIDERED TO BE RELEVANT**

| Category* | Citation of document, with indication, where appropriate, of the relevant passages | Relevant to claim No. |
|---|---|---|
| | CN 112175842 A (SHANGHAI HANHE BIOLOGICAL NEW MATERIAL TECHNOLOGY CO., LTD. et al.) 05 January 2021 (2021-01-05) | |
| Y | See abstract; and claims 1 and 5. | 11-18 |
| A | | 1-10 |
| | KIM, S.-J. et al. Screening and Characterization of an Enzyme with β-Glucosidase Activity from Environmental DNA. Journal of Microbiology and Biotechnology. 2007, vol. 17, no. 6, pp. 905-912. | |
| Y | See abstract; and page 909, right column. | 11-18 |
| | IMAMOGLU, S. Simulated Moving Bed Chromatography (SMB) for Application in Bioseparation. Advances in Biochemical Engineering Biotechnology. 2002, vol. 76, pp. 211-231. | |
| Y | See pages 225-226, and paragraph '4.1 Separation of Sugars'. | 17-18 |
| | KR 10-2019-0142480 A (KOOKMIN UNIVERSITY INDUSTRY ACADEMY COOPERATION FOUNDATION) 27 December 2019 (2019-12-27) | |
| A | See entire document. | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 March 2023** | **08 March 2023** |
| Name and mailing address of the ISA/KR<br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2022)

**INTERNATIONAL SEARCH REPORT**

International application No.
**PCT/KR2022/019160**

**C. DOCUMENTS CONSIDERED TO BE RELEVANT**

| Category* | Citation of document, with indication, where appropriate, of the relevant passages | Relevant to claim No. |
|---|---|---|
| A | US 2014-0302587 A1 (CHAABANE, F. B. et al.) 09 October 2014 (2014-10-09) See entire document. | 1-18 |

Form PCT/ISA/210 (second sheet) (July 2022)

**INTERNATIONAL SEARCH REPORT**
**Information on patent family members**

International application No.
**PCT/KR2022/019160**

| Patent document cited in search report | Publication date (day/month/year) | Patent family member(s) | Publication date (day/month/year) |
|---|---|---|---|
| CN 112175842 A | 05 January 2021 | CN 112175842 B | 27 December 2022 |
| KR 10-2019-0142480 A | 27 December 2019 | KR 10-2155049 B1 | 11 September 2020 |
| US 2014-0302587 A1 | 09 October 2014 | AU 2012-298391 A1 | 30 January 2014 |
| | | AU 2012-298391 B2 | 04 January 2018 |
| | | BR 112014003279 A2 | 01 March 2017 |
| | | BR 112014003279 B1 | 15 June 2021 |
| | | CA 2840539 A1 | 28 February 2013 |
| | | CA 2840539 C | 14 January 2020 |
| | | CN 103890171 A | 25 June 2014 |
| | | CN 103890171 B | 01 June 2016 |
| | | DK 2744899 T3 | 30 May 2016 |
| | | EP 2744899 A1 | 25 June 2014 |
| | | EP 2744899 B1 | 24 February 2016 |
| | | ES 2571459 T3 | 25 May 2016 |
| | | FR 2979111 A1 | 22 February 2013 |
| | | FR 2979111 B1 | 01 May 2015 |
| | | JP 2014-521359 A | 28 August 2014 |
| | | JP 6169077 B2 | 26 July 2017 |
| | | MX 2014000665 A | 14 November 2014 |
| | | MX 349345 B | 25 July 2017 |
| | | MY 168654 A | 28 November 2018 |
| | | PL 2744899 T3 | 31 August 2016 |
| | | UA 112783 C2 | 25 October 2016 |
| | | WO 2013-026964 A1 | 28 February 2013 |
| | | ZA 201400138 B | 30 September 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210168329
- KR 1020210168330


### Non-patent literature cited in the description


- **ANTONOV et al.** *Microb Cell Fact,* 2016, vol. 15, 164
- *APPLIED AND ENVIRONMENTAL MICROBIOLOGY,* January 1992, 106-110
- *Process Biochemistry,* 2001, vol. 37, 241-245